# EUROPÄISCHE PATENTSCHRIFT

(11) **EP 1 922 035 B1**
(45) Veröffentlichungstag und Bekanntmachung des Hinweises auf die Patenterteilung: **22.01.2014**
(21) Anmeldenummer: 06791866.4
(22) Anmeldetag: 06.09.2006
(51) Int. Cl.: A61F 2/46

(54) **EINSETZINSTRUMENT FÜR EINE ENDOPROTHESE MIT EINEM IN EINEM MARKRAUM EINZUSETZENDEN PROTHESENSCHAFT**
INSERTION INSTRUMENT FOR AN ENDOPROSTHESIS COMPRISING A PROSTHESIS SHAFT WHICH IS TO BE INSERTED INTO A MARKED CHAMBER
INSTRUMENT D'INSERTION POUR ENDOPROTHESE COMPORTANT UNE TIGE DE PROTHESE A INTRODUIRE DANS UN ESPACE MEDULLAIRE

(30) Priorität: 09.09.2005 DE 202005014270 U
(43) Veröffentlichungstag der Anmeldung: 21.05.2008
(73) Patentinhaber: WALDEMAR LINK GmbH & Co. KG, 22339 Hamburg (DE)
(72) Erfinder: KELLER, Arnold, 23863 Kayhude (DE)
(74) Vertreter: Thomas, Götz
(86) Internationale Anmeldenummer: PCT/EP2006/008679
(87) Internationale Veröffentlichungsnummer: WO 2007/028588

(56) Entgegenhaltungen:
- EP-A- 0 485 065
- DE-B- 2 101 002
- FR-A1- 2 839 641
- US-A- 4 936 863
- US-B1- 6 322 564

## Beschreibung

Die Erfindung betrifft ein Einsetzinstrument für eine Endoprothese gemäß dem Oberbegriff des Anspruchs 1.

Zur Verbindung von Endoprothesen mit dem Ende eines Röhrenknochens, wie dem Femur, sind üblicherweise Prothesesenschäfte vorgesehen, die in einen Markraum des Röhrenknochens eingeführt werden. Dazu ist bei der Implantation der Prothese der Markraum zu öffnen, sein Inneres in dem erforderlichen Maß zu räumen und schließlich den Schaft der Prothese einzusetzen. Um zu einem festen und sicheren Sitz des Prothesenschafts in den Röhrenknochen zu gelangen, soll die in dem Markraum zu schaffende Höhlung möglichst genau an die Kontur des aufzunehmenden Schafts angepasst sein. Nachträgliche Korrekturen der Position des Prothesenschafts führen zu Aufweitungen der Höhlung, und damit zu einem weniger sicheren Sitz. Die Langzeitstabilität der Prothese ist damit gefährdet. Dies gilt insbesondere dann, wenn die Implantation der Endoprothese zementlos erfolgen soll.

Es sind verschiedene Instrumente bekannt geworden, mit denen die Prothese mit ihrem Schaft implantiert werden kann. Aus der EP-B-0 852 931 ist ein Einsatzinstrument bekannt, das ein Griffteil mit einer Kupplung aufweist, an die eine Raspel zum Freiräumen der Höhlung im Markraum oder ein Schaft der einzusetzenden Endoprothese angeordnet werden kann. Die Raspel ist als Formraspel ausgebildet und dient dazu, eine der Kontur des einzusetzenden Schafts entsprechende Höhlung auszubilden. Ist die Höhlung geschaffen, so kann die Raspel von dem Griffstück abgenommen werden und nunmehr der Schaft der Endoprothese mit dem Griffteil verbunden werden. Das Griffteil fungiert nun als Einsetzinstrument. Ein ähnliches Einsetzinstrument ist aus der EP-B-0 956 824 bekannt. Das Griffteil weist eine fernbedienbare Schnellkupplung auf, mittels der die Raspel bzw. der einzusetzende Prothesenschaft an dem Griffteil gehalten ist. Weiter weist das Griffteil an seinem hinteren, kupplungsfernen Ende einen Amboss auf. Dieser dient als Schlagkopf. Damit kann bei straffem Sitz des Schafts in dem Markraum die Endoprothese durch Hammerschläge in ihre endgültige Position getrieben werden.

Ein Nachteil der bekannten Einsetzinstrumente liegt darin, dass Positionsveränderungen, wie sie insbesondere bei Korrekturen auftreten, zu einer Aufweitung und damit zu einer Verschlechterung des Sitzes des Schafts in dem Markraum des Knochens führen. Dies gilt insbesondere dann, wenn Korrekturen erst beim Einschlagen vorgenommen werden. Dazu kann es auch ungewollt kommen, wenn auf Grund schwieriger Zugangsbedingungen die Hammerschläge aus ungünstiger Richtung auf den Schlagkopf treffen. Dadurch kommt es nicht nur zu Fehlpositionierungen des Schafts und damit der Prothese in Bezug auf den Knochen, sondern auch zu einem schlechteren Sitz und damit zu einer Gefahr vorzeitiger Lockerung.

Aus der FR-A1-2 839 641 ist bereits ein Einsetzinstrument der eingangs genannten Art bekannt, bei welchem die Spitze des Stößels mit einem Außengewinde und die Endoprothese mit einer der Spitze des Stößels gegenüberliegenden Innengewindebohrung versehen ist, in welche die Stößelspitze zum Einschlagen der Endoprothese eingeschraubt wird. Die US-A-4 936 863 offenbart eine ähnliche Kombination aus Endoprothese und Einsetzinstrument. Jedoch gibt es eine Vielzahl von Endoprothesen ohne eine solche Innengewindebohrung, für welche die bekannten Einsetzinstrumente nicht ohne weiteres geeignet sind.

Die DE 21 01 002 B offenbart ein Einsetzinstrument für eine Endoprothese, das mit einem überstehenden Zapfen zum Einführen in eine Öse der Endoprothese versehen ist.

Aus der US-B1-6 322 564 ist ein Einsetzinstrument für eine Endoprothese bekannt, das keine Schlageinrichtung umfasst.

Die EP-A-0 485 065 offenbart ein prothetisches Hüftschaft-Implantat mit einem Drehmoment-Adapter, das mit einem nicht näher beschriebenen Stempel eingeschlagen wird.

Der Erfindung liegt die Aufgabe zu Grunde, ein Einsatzinstrument der eingangs genannten Art dahingehend zu verbessern, dass es ein sicheres und positionsgenaues Einsetzen vereinfacht.

Die erfindungsgemäße Lösung liegt in den Merkmalen des unabhängigen Anspruchs. Vorteilhafte Weiterbildungen sind Gegenstand der abhängigen Ansprüche.

Bei einem Einsetzinstrument für eine Endoprothese, die einen Halsteil und einen in einen Markraum einzusetzenden Schaftteil aufweist, umfassend eine Spanneinrichtung mit einer Haltegabel und einem Gegenlager, die dazu ausgebildet sind, in einem gespannten Zustand die Endoprothese festzuhalten und einem entspannten Zustand freizugeben, und eine Schlageinrichtung, bei dem an der Spanneinrichtung eine Führungsschiene für ein Schlagelement fest angeordnet und so ausgerichtet ist, dass ein von der Führungsschiene bestimmter Pfad auf eine Stirnseitenfläche der in der Spanneinrichtung gehaltenen Endoprothese führt und bei dem das Schlagelement ein in die Führungsschiene eingesetzter Stößel ist, ist erfindungsgemäß ein Rückspringdämpfer für den Stößel vorgesehen.

zuerst werden einige Begriffe erläutert:
Unter eine Haltegabel wird eine Aufnahme verstanden, die zu einer Seite hin offen ist, um den gehaltenen Gegenstand in die Aufnahme einzubringen oder aus ihr zu entnehmen. Die Öffnung bei eingebrachtem Gegenstand durch eine Verriegelung geschlossen sein, erforderlich ist dies aber nicht.

Unter einem Gegenlager wird ein zur Übertragung von Druckkräften ausgebildetes Element verstanden, das der Haltegabel gegenüberliegend so angeordnet ist, dass sie den zu haltenden Teil der Endoprothese zwischen sich spannen.

Unter einer Führungsschiene wird eine Anordnung verstanden, die dem Schlagelement sowohl eine Position wie auch eine Bewegungsrichtung (Pfad) vorgibt.

Die Erfindung beruht auf dem Gedanken, ein positionsgenaues Einsetzen des Schaftteils der Prothese in den Markraum mit dem Einsatzinstrument derart zu erreichen, dass die Position der Prothese sowohl beim Einführen, wie auch beim Einschlagen genau definiert ist. Mit dem erfindungsgemäßen Einsatzinstrument wird die genaue Führung der Prothese nicht nur in der Phase des Einführens in den Markraum sichergestellt, sondern auch darüber hinaus während der Phase des Einschlagens. Mit der an dem erfindungsgemäßen Einsetzinstrument angeordneten Führung für das Schlagelement wird erreicht, dass die Schlagkraft in genau definierter Weise auf die zu implantierende Prothese ausgeübt wird. Fehlpositionierungen, die durch unvermeidliche Variationen eines ungeführten Schlaginstruments, wie einen frei in der Hand geführten Hammer, entstehen können, werden wirkungsvoll vermieden. Dies ist insbesondere dann ein großer Vorteil, wenn die Zugänglichkeit des Implantationsortes für den Operateur eingeschränkt ist. Gerade unter erschwerten Zugangsbedingungen kommt es herkömmlicherweise auf Grund der dann nicht in exakter Verlängerung des Halterinstruments möglichen Führung des Schlaginstruments zu Abweichungen, die zu Fehlpositionierungen führen. Mit dem erfindungsgemäß ausgeführten Einsatzinstrument kann auch unter solch erschwerten Bedingungen eine positionsgenaue Implantation auf einfache und sichere Weise erreicht werden.

Durch die Masseverteilung des Stößels ist das Einsetzinstrument gut ausbalanciert. Um diese für die Handhabung günstige Eigenschaft auch beim Eintreiben der Prothese unter Nutzung des Stößels beizubehalten, ist der Stößel erfindungemäß mit einem Rückstoßdämpfer versehen. Dieser dient dazu, ein Zurückspringen des Stößels nach dem Schlag zu verhindern oder zumindest zu verringern. Ein solches Zurückspringen würde wie ein Rückschlag wirken, und zu der Gefahr einer unerwünschten Positionsänderung des Einsetzinstruments führen. Dem wird durch den erfindungsgemäßen Rückstoßdämpfer begegnet. Der für den Rückstoßdämpfer erforderliche Zusatzaufwand ist äußerst gering. So kann es bereits genügen, an dem Schaft des Stößels ein Reibelement vorzusehen, dass eine Reibungskraft auf die Führungsschiene ausübt. Besonders zweckmäßig ist es, an dem Schaft des Stößels eine Umfangsnut auszubilden, in die ein O-Ring eingesetzt ist. Die Abmessungen des O-Rings sind so gewählt, dass er innenseitig an der Wandung der als Rohr ausgeführten Führungsschiene anliegt.

Obgleich nicht unbedingt erforderlich, so ist es aber doch zweckmäßig, wenn die Führungsschiene seitlich geschlossen ist. Besonders bevorzugt ist es, wenn sie als ein Rohr ausgeführt ist. Sie kann dann zugleich als ein Teil des Handgriffs zum Greifen durch den Operateur fungieren. Die geschlossene Ausführung verhindert das Eintreten von Fremdkörpern. Die Gefahr einer Hemmung oder des Festgehens des Schlagelements in der Führung wird dadurch nahezu eliminiert.

Erfindungsgemäß ist das Schlagelement ein in die Führungsschiene eingesetzter Stößel. Der Stößel ist auf Grund seiner langgestreckten Form in der vorzugsweise als Rohr mit konturgleicher Innenform ausgeführten Führungsschiene optimal geführt. Der Schaft des Stößels dient aber nicht nur zur Führung, sondern fungiert auf Grund seiner durch die verhältnismäßig lange Erstreckung nicht unbeträchtlichen Masse selbst auch bereits als Schlagelement. Ein optional zusätzlich am Ende des Stößels vorgesehener Schlagkopf kann daher kleiner ausgeführt sein. Das Einsetzinstrument kommt dadurch mit verhältnismäßig geringem Zusatzgewicht aus, und ist auf Grund der verteilten Masse des Stößels in handhabungstechnisch günstiger Weise ausbalanciert. Der Schlagkopf ist vorzugsweise als Handhabe ausgeführt. Damit kann der Stößel sowohl von Hand betätigt werden, wie auch über seine als Ambossfläche ausgebildete Rückseite als Schlagkopf verwendet werden.

Der Stößel weist an seinen vorderen Enden eine Spitze auf, die mit Vorteil abgerundet ist. Gegenüber eine stumpfen Spitze ergibt sich hiermit der Vorteil, dass bei einem winkligen Auftreffen auf eine Stirnfläche der Endoprothese die Gefahr von Einkerbungen und Beschädigungen verringert ist. Einkerbungen oder andere Beschädigungen würden zu einer Schwächung des Prothesenmaterials an dieser Stelle führen, was zu entsprechender Verringerung der Stabilität und damit der Langzeithaltbarkeit der Prothese führen kann. Die abgerundete Ausführung der Spitze bietet auch einen Vorteil in dem Fall, wenn die den Stößelschlag aufnehmende Stirnfläche der Prothese nicht ganz eben ist.

Der Stößel ist mit Vorteil segmentiert ausgeführt, wobei ein Segment einen kleineren Querschnitt als das andere aufweist. Liegt das Segment mit dem kleineren Querschnitt in Richtung auf die Halteeinrichtung gesehen vorne, so kann mit der sich am Übergang zu dem Segment mit dem breiteren Querschnitt ergebenen Schulter ein Tiefenanschlag für den Stößel ausgebildet sein. Die Gefahr eines Herausrutschens und zu weitem Heraustreibens des Stößels zu der zu implantierenden Endoprothese hin ist dadurch minimiert. Die sich damit ergebene stufige Verjüngung des Schafts zur Spitze ermöglicht nicht nur die Bildung eines Anschlags, sondern erleichtert auch das Auswechseln des O-Rings von der Spitze des Stößels her.

Gemäß einem weiteren Aspekt der Erfindung ist an der Spanneinrichtung ein Positionsmarker für eine winkelrichtige Lage der Endoprothese an dem Einsetzinstrument vorgesehen. Damit steht dem Operateur eine einfach zu benutzende Anzeige dafür zur Verfügung, dass die Prothese in der richtigen Winkelorientierung an dem Einsetzinstrument gehaltert ist. Der Positionsmarker kann auf verschieden Weise ausgeführt sein, im einfachsten Fall durch Markierungen. Es hat sich bewährt, die Positionsmarkierungen als Vorsprünge an der Spanneinrichtung auszuführen, die zum Eingreifen in einen Rezess an der Endoprothese im gespannten Zustand ausgebildet sind. Rasten die Vorsprünge nur in der winkelrichtigen Lage in die jeweiligen Rezesse an der Endoprothese ein, so kann auf einfache und zweckmäßige Weise die Endoprothese in ihrer relativen Lage zu dem Einsetzinstrument fixiert sein. Eine visuelle Kontrolle der Orientierung ist überflüssig. Die Orientierung bleibt auch unter erschwerten Umständen bei schlechter Zugänglichkeit vorhanden, oder kann gegebenenfalls auch ohne Blickkontakt leicht wieder hergestellt werden. Bevorzugterweise ist dabei der Vorsprung an dem Gegenlager angeordnet und so bemessen, dass er etwa 0,1 mm bis 1 mm größer ist als die Tiefe des Rezesses in einer Stirnseite der Endoprothese. Damit wird erreicht, dass der Kontakt zwischen dem Gegenlager und der Endoprothese über die Vorsprünge läuft, genauer gesagt, dass die Spitzen der Vorsprünge am Grund der Rezesse aufliegen. Das Gegenlager braucht im Übrigen keinen Kontakt mit der Endoprothese zu haben, insbesondere kann das Gegenlager frei bleiben von der Stirnseite der Endoprothese. Dies ist ein nicht zu unterschätzender Vorteil, da nicht selten die Stirnseite der Endoprothese Teil eines maßhaltigen Halteteils ist, das zur Gewährleistung maßgenauer Positionierung von Gelenkverbindungskomponenten möglichst vor Beschädigungen geschützt sein muss.

Bei einer zweckmäßigen Ausführungsform ist die Haltegabel derart ausgebildet, dass in ihr ein Sitz für einen Befestigungskonus des Halteteils eingearbeitet ist. In dem Sitz ist der Befestigungskonus mit seinem unteren Rand in der Haltegabel aufgenommen worden. Der Sitz braucht dabei keine große Tiefe aufzuweisen, eine Tiefe von bis zu 2,5 mm, vorzugsweise 1 mm, genügt regelmäßig. Mit diesem Sitz wird erreicht, dass die Haltegabel nur in dem unteren Randbereich auf den Befestigungskonus einzuwirken braucht. Die empfindliche Konusmantelfläche bleibt damit vor Einwirkungen durch die Haltegabel verschont. Beschädigungen der Mantelfläche können auf diese Weise verhindert werden.

Die Erfindung wird nachfolgend unter Bezugnahme auf die beigefügte Zeichnung näher beschrieben, in der ein Ausführungsbeispiel dargestellt ist. Es zeigen:

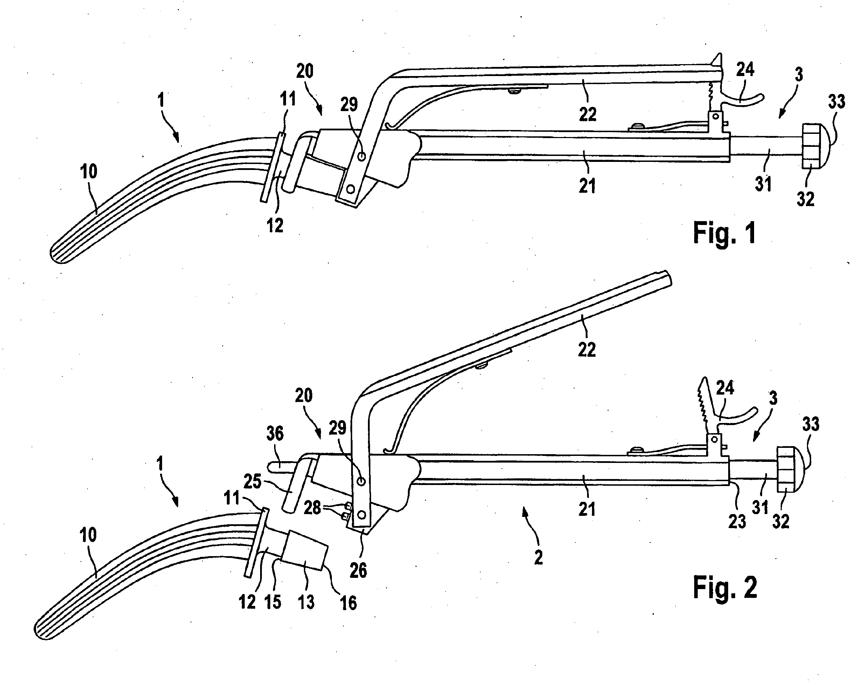

- Fig. 1: eine Aufsicht auf ein Ausführungsbeispiel für ein erfindungsgemäßes Einsetzinstrument im gespannten Zustand mit einer Endoprothese;
- Fig. 2: eine Ansicht des Einsetzinstruments gemäß Figur 1 im entspannten Zustand;

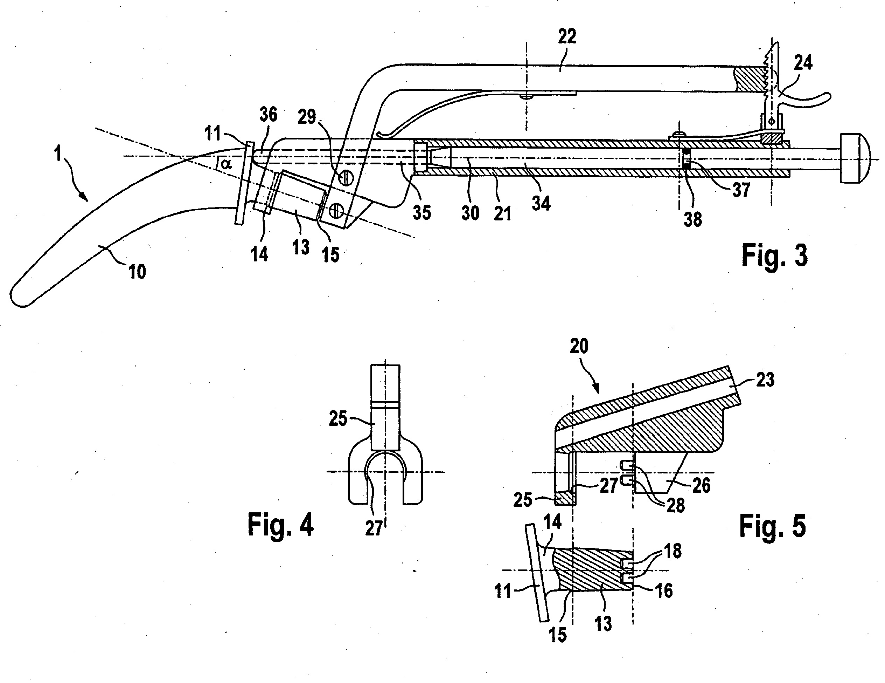

- Fig. 3: einen Teilschnitt durch das in Figur 1 dargestellte Einsetzinstrument;
- Fig. 4: eine Frontalansicht eines Teils einer Spanneinrichtung des in Figur 1 dargestellten Einsetzinstruments; und
- Fig. 5: eine Teilschnittansicht der Spanneinrichtung mit der aufzunehmenden Endoprothese.

Das Einsetzinstrument gemäß dem Ausführungsbeispiel ist von generell zangenartiger Grundgestalt mit einem Griffteil im hinteren Bereich und einem Spannteil (nachfolgend als Spanneinrichtung bezeichnet) 29 im vorderen Bereich. Die beiden Bereiche sind getrennt durch ein Schwenkgelenk 29. Der Griffteil weist 2 stabartig sich nach hinten erstreckende Handgriffe 21, 22 auf. Sie sind in einer zusammengedrückten Stellung mittels einer Rasteinrichtung 24 fixierbar.

An dem vorderen Ende des Einsetzinstruments, das in seiner Gesamtheit mit dem Bezug zu Ziffer 2 bezeichnet ist, ist die Spanneinrichtung 20 angeordnet.'Sie besteht im Wesentlichen aus einer Gabel 25 am vorderen Ende des mit dem Handgriff 21 verbundenen Schenkels. Die Gabel 25 weist eine Aufnahmeöffnung auf, in welche die zu spannende Endoprothese 1 einzusetzen ist. Die Spanneinrichtung 20 weist weiter an dem vorderen Ende des von dem Handgriffs 22 gebildeten Schenkels ein Gegenlager auf. Es ist gebildet durch einen Gleitblock 26, der schwenkbar an dem vorderen Ende angeordnet ist, so dass er eine gleitende Vor- und Zurückbewegung beim Schließen und Öffnen der Spanneinrichtung 20 ausführt ist. Der Gleitblock 26 weist eine der Haltegabel 25 zugewandte Stirnseite auf, an der zwei Vorsprünge 28 angeordnet sind. Die Vorsprünge sind stiftartig ausgeführt und weisen eine Länge von etwa 4 mm auf. Sind so orientiert, dass sie auf die Haltegabel 25 gerichtet sind, und zwar deren Öffnungsbereich.

Es wird nun der Aufbau der Führungsschienen bei dem Einsetzinstrument beschrieben. Der Handgriff 21 ist in seinem hinteren Bereich rohrförmig ausgeführt. Er weist eine sich durch seine gesamte Länge erstreckende Bohrung 23 auf. Sie fungiert als Führungsschiene für einen Stößel 31. Der Stößel 31 ist als Schlagwerkzeug ausgebildet und besteht im Wesentlichen aus seinem in zwei Segmente 34, 35 unterteilten Schaft mit einer abgerundeten Spitze 36. An dem hinteren Ende des Stößels 31 ist eine Handhabe 32 zur Betätigung durch den Operateur angeordnet. Der Stößel 31 besteht wie das übrige Einsetzinstrument aus Edelstahl. Auf Grund der Masse des Stößels 31 einschließlich der Handhabe 32 kann in vielen Fällen durch Handkraft eine ausreichende Schlagwirkung erzielt werden. Für den Fall, dass eine höhere Schlagwirkung erforderlich ist, ist die Rückseite der Handhabe 32 mit einer Ambossfläche 33 versehen. Auf sie kann mit Hammerschlägen eingewirkt werden, wodurch die von der Spitze 36 übertragene Schlagwirkung deutlich gesteigert werden kann.

An dem Stößel 31 ist an seiner Außenfläche im Bereich des ersten Segments 34 eine Umfangsnut 37 ausgebildet. In ihr ist ein O-Ring 38 angeordnet. Er ist so bemessen, dass er nicht bündig mit der Oberfläche des Schaftsegments 34 abschließt, sondern soweit heraussteht, dass er die Innenwandung der Bohrung 31 kontaktiert. Dadurch übt der O-Ring eine Reibkraft aus, die sich der Bewegung des Stößels 31 entgegen richtet. Der O-Ring 38 kann damit ein Rückspringen des Stößels 31 nach Schlageinwirkung, insbesondere bei Hammerschlägen auf die Ambusfläche 33, weitgehend verhindern.

Zur Erläuterung der Wirkungsweise des Einsatzinstruments wird nachfolgend kurz die zu implantierende Endoprothese vorgestellt. Bei der Endoprothese handelt es sich eine Schaftprothese, wie sie die Femurkomponente einer totalen Hüftgelenksprothese bildet. Die Schaftprothese besteht im Wesentlichen aus einem lang gestreckten gebogenen Schaftteil 10, einem Auflagekragen 11 und einem sich auf dem Auflagekragen 11 erhebenden Halsteil 14. In dem äußeren Bereich des Halsteils 14 ist ein Konus 13 ausgebildet. Er dient zur Aufnahme einer Gelenkkugel. Die Mantelfläche des Konus 13 sowie seine Stirnseite 16 definieren dabei die Positionierung der Gelenkkugel (nicht dargestellt). Sie sind präzise gefertigt und müssen vor Beschädigungen geschützt werden. Der Schaftteil 10 dient dazu, in den Markraum des (zuvor geöffneten) Femurs implantiert eingesetzt zu werden. Der Schaftteil 10 kann zur zementierten oder zementfreien Implantation ausgebildet sein. Gerade im Fall der zementfreien Implantation ist ein guter Formschluss zu der umgebenden Struktur im Markraum wichtig. Richtungsveränderungen der Endoprothese 1 bzw. der Schaftteil 10 während des Einsetzens oder des Einschlagens führen zu Aufweitungen im Markraum, wodurch die Formschlüssigkeit beeinträchtigt wird und damit die Stabilität und Sicherheit der Verankerung der Endoprothese in dem Femurknochen verringert wird. Um eine sichere Führung der Endoprothese 1 beim Einsetzen und Einschlagen zu ermöglichen, ist sie fest an dem Einsetzinstrument 2 eingespannt. Dies geschieht mit der Spanneinrichtung 20 in der nachfolgend beschriebenen Weise.

Die Spanneinrichtung 20 mit ihrer Haltegabel 25 und ihrem Gegenlager 26 ist dazu ausgebildet, die Endoprothese 1 an den Konus 13 des Halsteils 14 zu halten. In der Haltegabel 25 ist eine kreisförmige Stufe eingearbeitet, die als Sitz 27 für den unteren Rand 15 des Konus 13 dient. Die Tiefe des Sitzes 27 ist so gewählt, dass der Konus 13 etwa 1 mm tief in die Haltegabel 25 eintaucht. Nur in diesem Bereich kommt es zu einem Kontakt zwischen der Haltegabel 25 und Konus 13. Dieser Bereich ist typischerweise für die Verbindung zum Gelenkkopf unkritisch, da die Gelenkköpfe sich nicht so weit auf den Konus 13 erstrecken. In dem übrigen Bereich des Konus 13 greift der Sitz 27 nicht an, so dass dieser vor Beschädigung geschützt ist. Das Gegenlager 26 wirkt mit der Stirnseite 16 des Konus 13 zusammen. Es liegt aber nicht direkt an der Stirnseite 16 an, sondern greift mit seinen Vorsprüngen 28 in entsprechende Ausnehmungen 18 an der Stirnseite 16 des Konus 13 ein. Dabei ist die Höhe der Vorsprünge 28 etwas größer als die Tiefe der Ausnehmungen 18. Bewährt hat sich eine Höhe von 4 mm für die Vorsprünge 28 bei einer Tiefe von 3 mm für die Ausnehmungen 18. Damit wird erreicht, dass die Vorsprünge 28 mit ihrer Spitze in dem Grund der Ausnehmungen 18 aufliegen, ohne dass die Stirnseite 16 selbst von dem Gleitblock 26 berührt wird. Es bleibt ein Sicherheitsspalt von ca. 1 mm. Der Gleitblock 26 mit den Vorsprüngen 28 wird durch Zusammendrücken der Griffteile 21 und 22 nach vorne (in Spannrichtung) bewegt. Dabei tauchen die Vorsprünge 28 in die Ausnehmungen 18 ein und pressen den Konus 13 mit seinem unteren Rand 15 in den Sitz 27 der Haltegabel 25. Damit ist die Endoprothese 1 fest an das Einsetzinstrument gespannt. Die Winkelposition ist durch die Vorsprünge 28 definiert. Sie fungieren damit auch als Positionsmarker. Die Prothese 1 kann von dem Operateur nun sicher und positionsgenau geführt werden.

Im ersten Schritt der Implantation wird die Prothese 1 mit ihrem Schaft 10 mittels des Einsetzinstruments 2 in den Markraum eingebracht. Dies gelingt über etwa 2/3 bis 3/4 der Länge des Schafts 10 wegen seiner Konizität meist ohne besonderen Kraftaufwand. Danach erfordert der zunehmend straffer werdende Sitz des Prothesenschafts 10 in dem Bett des Markraums eine immer größere Kraft, mit der bis zum Erreichen der endgültigen Position die Endoprothese 1 eingeschlagen werden muss. Dies kann mit dem erfindungsgemäßen Einsetzinstrument geschehen, ohne dass dazu das Instrument abgesetzt zu werden braucht. Der Operateur betätigt mittels der Handhabe 32 den Stößel 31. Die Anordnung der Haltegabel 25 mit dem Gegenlager 26 ist dabei derart auf die Lage der Bohrung 23 abgestimmt, dass der von ihr bestimmte Pfad 30 der Stößels so gewählt ist, dass die Spitze 36 an dem Kragen 11 auf die Endoprothese 1 trifft. Der von dem Stößel 31 übertragene Impuls wird damit direkt auf den Kragen 11 und damit auf den Schaft 10 geleitet, ohne den Konus 13 und den Halsteil 14 zu belasten. Diese direkte Übertragung ist nicht nur schonend für den Konus 13 und den Halsteil 14 der Endoprothese 1, sondern ermöglicht es, auch mit verhältnismäßig schwachen Schlägen durch den Stößel 31 eine hohe eintreibende Wirkung zu erzielen.

Der Winkel der Anordnung der Endoprothese bezogen auf den Pfad 30 des Stößels (Winkel α) beträgt etwa 20 Grad bis 40 Grad (vorzugsweise 25 Grad) und ist so gewählt, dass in der Position der Endoprothese 1, in der die höchsten Kräfte zum Einschlagen erforderlich, der Führungspfad 30 etwa senkrecht zur Sektionsfläche an dem Knochen liegt. Damit ist für diesen besonders hohen Kraftaufwand erfordernden Abschnitt eine optimale Kraftübersetzung sicher gestellt.

Im dem erfindungsgemäßen Einsetzinstrument kann die Endoprothese positions- und winkelgenau an das Einsetzinstrument gespannt werden, und in dieser wohldefinierten Lage präzise in den Markraum eingeschoben werden. Das zum Erreichen der festen Endposition der Prothese erforderliche Nachschlagen kann mit dem Einsetzinstrument durchgeführt werden, wobei durch die Führung des Stößels 31 sichergestellt ist, dass die Impulskräfte durch das Schlagen an eine günstige Stelle an dem Kragen 11 der Endoprothese 1 eingeleitet werden. Der empfindliche Konus 13 der Endoprothese 1 ist dabei vor der Aufnahme von Schlagkräften geschützt. Das Einschlagen kann unter Nutzung des integrierten Stößels 31 ohne Instrumentenwechsel erfolgen. Erforderlichenfalls kann über die Ambusfläche 33 am hinteren Ende des Stößels 31 zusätzlich mit einem Hammer nachgeschlagen werden.

## Patentansprüche

1. Einsetzinstrument für eine Endoprothese, die einen Halsteil (14) und einen in einem Markraum einzusetzenden Schaftteil (10) aufweist, umfassend eine Spanneinrichtung (20) mit einer Haltegabel (25) und einem Gegenlager (26), die dazu ausgebildet sind, in einem gespannten Zustand die Endoprothese (1) festzuhalten und einem entspannten Zustand freizugeben, und eine Schlageinrichtung (3), wobei an der Spanneinrichtung (20) eine Führungsschiene (23) für ein Schlagelement fest angeordnet ist und so ausgerichtet ist, dass ein von der Führungsschiene bestimmter Pfad auf eine Stirnseitenfläche der in der Spanneinrichtung gehaltenen Endoprothese führt, und wobei das Schlagelement ein in die Führungsschiene (23) eingesetzter Stößel (31) ist, **dadurch gekennzeichnet, dass** ein Rückspringdämpfer für den Stößel (31) vorgesehen ist.

2. Einsetzinstrument nach Anspruch 1,
**dadurch gekennzeichnet, dass**
die Führungsschiene (23) geschlossen ist.

3. Einsetzinstrument nach Anspruch 2,
**dadurch gekennzeichnet, dass**
die Führungsschiene (23) als Rohr ausgeführt ist.

4. Einsetzinstrument nach einem der vorhergehenden Ansprüche,
**dadurch gekennzeichnet, dass**
in die Haltegabel (25) ein Sitz (27) für einen Befestigungskonus (13) der Prothese eingearbeitet ist.

5. Einsetzinstrument nach Anspruch 4,
**dadurch gekennzeichnet, dass**
der Sitz (27) eine Tiefe von weniger 2 mm, vorzugsweise weniger als 1 mm aufweist.

6. Einsetzinstrument nach Anspruch 5,
**dadurch gekennzeichnet, dass**
der Stößel (31) an seinem hinteren, endoprothesenfernen Ende eine Handhabe (32) aufweist.

7. Einsetzinstrument nach Anspruch 6,
**dadurch gekennzeichnet, dass**
die Handhabe (32) eine Schlagfläche (33) aufweist.

8. Einsetzinstrument nach einem der Ansprüche 5 bis 7,
**dadurch gekennzeichnet, dass**
der Stößel (31) eine gerundete Spitze (36) aufweist.

9. Einsetzinstrument nach Anspruch 8,
**dadurch gekennzeichnet, dass**
der Rückspringdämpfer als ein in einer Umfangsnut (37) angeordneter O-Ring (38) ausgeführt ist.

10. Einsetzinstrument nach einem der Ansprüche 5 bis 9,
**dadurch gekennzeichnet, dass**
der Stößel (31) segmentiert ausgeführt, wobei ein Segment (35) einen kleineren Querschnitt als das andere Segment (34) aufweist.

11. Einsetzinstrument nach einem der vorangehenden Ansprüche,
**dadurch gekennzeichnet, dass**
an der Spanneinrichtung (20) Positionsmarken für eine winkelrichtige Lage der Endoprothese vorgesehen sind.

12. Einsetzinstrument nach Anspruch 11,
**dadurch gekennzeichnet, dass**
die Positionsmarke ein Vorsprung (28) an der Spanneinrichtung (20) ist, der zum Eingreifen in einem Rezess (18) an der Endoprothese (1) im gespannten Zustand ausgebildet ist.

13. Einsetzinstrument nach Anspruch 12,
**dadurch gekennzeichnet, dass**
der Vorsprung (28) an dem Gegenlager (26) angeordnet ist und vorzugsweise 0,1 mm bis 1 mm größer ist als die Tiefe des Rezesses (18).

## Claims

1. Insertion instrument for an endoprosthesis, the latter having a neck part (14) and a shaft part (10) that is to be inserted into a medullary cavity, the insertion instrument comprising a clamping mechanism (20) with a holding fork (25) and a thrust bearing (26), which are designed to firmly hold the endoprosthesis (1) in a clamped state and to release it in an unclamped state, and a striking mechanism (3), wherein a guide rail (23) for a striking element is arranged in a fixed manner on the clamping mechanism (20) and is oriented such that a path determined by the guide rail leads to an end face of the endoprosthesis held in the clamping mechanism, and wherein the striking element is a ram (31) inserted into the guide rail (23), **characterized in that** a recoil damper is provided for the ram (31).

2. Insertion instrument according to Claim 1, **characterized in that** the guide rail (23) is closed.

3. Insertion instrument according to Claim 2, **characterized in that** the guide rail (23) is designed as a tube.

4. Insertion instrument according to one of the preceding claims, **characterized in that** a seat (27) for a securing cone (13) of the prosthesis is worked into the holding fork (25).

5. Insertion instrument according to Claim 4, **characterized in that** the seat (27) has a depth of less than 2 mm, preferably of less than 1 mm.

6. Insertion instrument according to Claim 5, **characterized in that** the ram (31) has a handle (32) at its rear end directed away from the endoprosthesis.

7. Insertion instrument according to Claim 6, **characterized in that** the handle (32) has a striking surface (33).

8. Insertion instrument according to one of Claims 5 to 7, **characterized in that** the ram (31) has a rounded tip (36).

9. Insertion instrument according to Claim 8, **characterized in that** the recoil damper is designed as an O-ring (38) arranged in a peripheral groove (37).

10. Insertion instrument according to one of Claims 5 to 9, **characterized in that** the ram (31) is segmented, wherein one segment (35) has a smaller cross section than the other segment (34).

11. Insertion instrument according to one of the preceding claims, **characterized in that** position markers for correct angular positioning of the endoprosthesis are provided on the clamping mechanism (20).

12. Insertion instrument according to Claim 11, **characterized in that** the position marker is a projection (28) on the clamping mechanism (20), which projection (28) is designed to engage in a recess (18) on the endoprosthesis (1) in the clamped state.

13. Insertion instrument according to Claim 12, **characterized in that** the projection (28) is arranged on the thrust bearing (26) and is preferably 0.1 mm to 1 mm greater than the depth of the recess (18).

## Revendications

1. Instrument d'insertion pour une endoprothèse qui présente une partie de col (14) et une partie de tige (10) devant être insérée dans un espace médullaire, comprenant un dispositif de serrage (20) avec une fourche de retenue (25) et un contre-palier (26), lesquels sont réalisés de manière à fixer l'endoprothèse (1) dans un état serré et à la libérer dans un état desserré, et un dispositif percuteur (3), un rail de guidage (23) pour un élément percuteur étant disposé fixement sur le dispositif de serrage (20) et étant orienté de telle sorte qu'un chemin déterminé par le rail de guidage conduise à une surface du côté frontal de l'endoprothèse retenue dans le dispositif de serrage, l'élément percuteur étant un poussoir (31) inséré dans le rail de guidage (23), **caractérisé en ce qu'**un amortisseur à ressort de rappel est prévu pour le poussoir (31).

2. Instrument d'insertion selon la revendication 1,
**caractérisé en ce que**
le rail de guidage (23) est fermé.

3. Instrument d'insertion selon la revendication 2,
**caractérisé en ce que**
le rail de guidage (23) est réalisé sous forme de tube.

4. Instrument d'insertion selon l'une quelconque des revendications précédentes,
**caractérisé en ce**
**qu'**un siège (27) pour un cône de fixation (13) de la prothèse est incorporé dans la fourche de retenue (25).

5. Instrument d'insertion selon la revendication 4,
**caractérisé en ce que**
le siège (27) présente une profondeur inférieure à 2 mm, de préférence inférieure à 1 mm.

6. Instrument d'insertion selon la revendication 5,
**caractérisé en ce que**
le poussoir (31) présente, au niveau de son extrémité arrière, éloignée de l'endoprothèse, une manette (32).

7. Instrument d'insertion selon la revendication 6,
**caractérisé en ce que**
la manette (32) présente une surface de percussion (33).

8. Instrument d'insertion selon l'une quelconque des revendications 5 à 7,
**caractérisé en ce que**
le poussoir (31) présente une pointe arrondie (36).

9. Instrument d'insertion selon la revendication 8,
**caractérisé en ce que**
l'amortisseur à ressort de rappel est réalisé sous forme de joint torique (38) disposé dans une rainure périphérique (37).

10. Instrument d'insertion selon l'une quelconque des revendications 5 à 9,
**caractérisé en ce que**
le poussoir (31) est réalisé sous forme segmentée, un segment (35) présentant une plus petite section transversale que l'autre segment (34).

11. Instrument d'insertion selon l'une quelconque des revendications précédentes,
**caractérisé en ce que**
des marques de positionnement pour une position angulaire correcte de l'endoprothèse sont prévues sur le dispositif de serrage (20).

12. Instrument d'insertion selon la revendication 11,
**caractérisé en ce que**
la marque de positionnement est une saillie (28) sur le dispositif de serrage (20), laquelle est réalisée pour venir en prise dans un retrait (18) sur l'endoprothèse (1) dans l'état serré.

13. Instrument d'insertion selon la revendication 12,
**caractérisé en ce que**
la saillie (28) est disposée sur le contre-palier (26) et présente une dimension de préférence d'environ 0,1 mm à 1 mm supérieure à la profondeur du retrait (18).
